# EUROPEAN PATENT SPECIFICATION

(11) **EP 2 775 963 B1**
(45) Date of publication and mention of the grant of the patent: **21.12.2016**
(21) Application number: 12784311.8
(22) Date of filing: 31.10.2012
(51) Int. Cl.: A61F 2/34

(54) **PROSTHESIS COMPONENT**
PROTHESENTEIL
COMPOSANT DE PROTHÈSE

(30) Priority: 03.11.2011 GB 201119017
(43) Date of publication of application: 17.09.2014
(73) Proprietor: Biomet UK Healthcare Limited, Bridgend South Wales CF31 3XA (GB)
(72) Inventor: BIGSBY, Robert John Andrew, Glamorgan South Wales CF64 1EJ (GB); KHAN (Dr), Mohammed Imran, Reading Berkshire RG1 3PP (GB)
(74) Representative: Mays, Julie
(86) International application number: PCT/GB2012/052701
(87) International publication number: WO 2013/064817

(56) References cited:
- EP-A1- 0 461 019
- EP-A2- 1 442 727
- WO-A1-96/22746
- WO-A1-2011/008752
- FR-A1- 2 795 302
- US-A- 6 042 612
- US-A1- 2011 251 698

## Description

The present invention relates to a prosthesis component, and particularly to a prosthesis component having a shell and articulating liner.

### Background

It is known to replace some or all of a natural bone joint that has become damaged or diseased with prosthetic components. For example, a natural hip joint that has become degraded may be replaced with an artificial joint comprising a prosthetic acetabular component which is implanted into the patient's acetabulum, and a prosthetic femoral head component which is implanted into the patient's femur and articulates with the acetabular component. A prosthetic joint of this type is known as a total hip replacement (THR).

Various types of THR exist and are well known in the art. The different types of prosthesis take different approaches to balancing the competing requirements of reducing wear in the joint, maintaining stability of the joint, increasing patient range of motion, and many other factors. Reducing wear on the prosthesis components can be achieved by reducing as far as possible the surface area over which the components articulate. This leads to the use of smaller diameter femoral head components, which have favourable wear characteristics but suffer from a limited range of motion. In contrast, in order to promote joint stability it is desirable to use a large diameter femoral head component, which has the additional benefit of a better range of patient motion but results in an increase in the articulating surface area, and hence risk of wear damage. One type of prosthetic hip joint that seeks to combine advantages of reduced wear with increased stability and range of motion is the so called "double mobility" total hip prosthesis. In a double mobility THR, a prosthetic femoral head of relatively small diameter is received within a polyethylene "liner" within which the head articulates. This articulation is the primary mobility of the joint, and benefits from the reduced wear associated with a smaller articulating surface. The second mobility of the joint arises from the movement of the polyethylene liner within an exterior metallic shell. This second articulation benefits from increased stability, owing to the relatively large external diameter of the liner. The large diameter of the second articulation, in addition to being highly stable, also offers a greater range of motion than the smaller first articulation. The second articulation only comes into operation at the extremes of motion of the joint, when the smaller primary articulation has reached its limit. The majority of motion within the joint thus takes place at the lower wearing primary articulation between the femoral head and the liner. By combining large and small diameter articulations in the same prosthesis, dual mobility prostheses offer some of the greatest ranges of motion available in THRs, as well as being some of the most stable of the prosthesis options available. In order to increase stability still further, it is known to use an additional circular clip, or circlip, to engage around the femoral neck and cooperate with the polyethylene liner and shell to maintain the femoral head within the liner. It is also known to provide the exterior metal shell with an additional superior lip, in order to support the polyethylene liner and prevent any possible creep deformation which may impede performance of the device.

While the double mobility type hip prosthesis has experienced good results, there remain some disadvantages to the system. The larger articulation of polyethylene liner against metal shell is excellent for stability, but it does not provide the best wear characteristics. Some prostheses have experimented with alternative metal alloys and material coatings for the shell to address the wear characteristics, but in the main this issue has been ignored in existing devices as the articulation is only engaged at extremes of motion and the wear performance of the articulation has not been considered a priority.

Fixation is another area where difficulties can arise with a double mobility prosthesis. The conventional means of fixation for the external metallic shell is to impact the shell into the prepared acetabulum, with the additional use of bone cement if required. This is acceptable in many cases but does not offer alternatives for patients with a high level of degradation of the acetabular bone tissue. In conventional single mobility hip prostheses, additional fixation may be provided by bone screws which pass through the outer shell and engage deeply into the patient's bone tissue to hold the shell in place. A static liner is then fitted into the shell, over the screw heads to provide the articulating surface for the femoral head. In the case of a double mobility prosthesis the liner must articulate against the shell, and so the inclusion of bone screws can only be achieved by increasing the degree of modularity within the device, adding an extra liner between the shell and the articulating liner. Such an additional stationary liner can be inserted to cover the screw heads and provide the articulating surface for the mobile liner. However, the inclusion of the extra liner reduces the space available for the femoral head, meaning the extra fixation comes at the expense of a reduced range of motion.

The present invention seeks to address some or all of the above mentioned issues. The features of the preambles of independent claims 1 and 17 are known from US-A-6 042 612.

### Summary of Invention

According a first aspect of the present invention, there is provided a prosthesis component comprising: a shell formed form a fibre reinforced polymer material and having an inner bearing surface; and a liner having an outer bearing surface formed from a ceramic material; wherein the liner is received in an articulating manner within the shell.

The shell may further comprise one or more openings on the inner bearing surface.

The one or more openings may extend through the thickness of the shell to open on an outer surface of the shell.

The openings may be suitable to receive one or more attachment means operable to secure the prosthesis component in place.

The prosthesis component may further comprise one or more attachment means, which may be operable to extend through the openings to secure the prosthesis component in place.

The attachment means may be formed from a fibre reinforced polymer material and may be formed from the same fibre reinforced polymer material as the shell.

The shell may be formed from a carbon fibre reinforced polymer material and may be formed from carbon fibre reinforced polyether ether ketone (CFR-PEEK).

A hydroxyapatite (HA) coating may be formed on an outer surface of the shell. Alternatively, or in addition, a porous metal coating may be formed on an outer surface of the shell.

The prosthesis component may be an acetabular prosthesis component.

The liner may be formed from a ceramic material.

The liner may further comprise an inner bearing surface which may be operable to receive a femoral head prosthesis component.

The prosthesis component may further comprise a retaining element, which may be operable to cooperate with the liner and the shell to retain a femoral head prosthesis component in engagement with the inner bearing surface of the liner.

The retaining element may be formed from a fibre reinforced polymer material, and may be formed from the same fibre reinforced polymer material as the shell. The material may be a carbon fibre reinforced polymer material.

The retaining element may comprise a circular clip, or circlip.

According to another aspect of the present invention, there is provided a hip replacement prosthesis comprising a prosthesis component according to the first aspect of the present invention and a femoral head prosthesis component.

The femoral head prosthesis component may be formed from a ceramic material or may be formed from a coated metallic material.

According to another aspect of the present invention, there is provided a kit of parts for a hip replacement prosthesis comprising: an acetabular shell formed from a fibre reinforced polymer material and comprising an inner bearing surface; an acetabular liner having an outer bearing surface formed from a ceramic material and operable to articulate with the inner bearing surface of the shell and an inner bearing surface; and a femoral head prosthesis having a bearing surface formed from a ceramic material and operable to articulate with the inner bearing surface of the liner.

The acetabular liner may be formed from a ceramic material, the acetabular shell may be formed from a carbon fibre reinforced polymer material and the femoral head prosthesis may be formed from a ceramic material.

The kit may further comprise a plurality of attachment means formed from a fibre reinforced polymer material. The material may be a carbon fibre reinforced polymer material.

The kit may further comprise a circular clip, or circlip, which may be operable to cooperate with the liner and the shell to retain the femoral head prosthesis in engagement with the inner bearing surface of the liner. The circlip may be formed from a carbon fibre reinforced polymer material.

There is also disclosed a prosthesis comprising a shell, a liner received in an articulating manner within the shell to form a first bearing couple, and a head received in an articulating manner within the liner to form a second bearing couple, wherein the bearing surfaces of the second bearing couple, and a bearing surface of the first bearing couple, are relatively harder than the other bearing surface of the first bearing couple.

For the purposes of the present specification, the phrase "bearing couple" refers to a pair of bearing surfaces that engage each other. The first bearing couple may operate under boundary lubrication. The bearing surfaces of the second bearing couple and a bearing surface of the first bearing couple may be formed from a ceramic material. The other bearing surface of the first bearing couple may be formed form a fibre reinforced polymer material, which may be a carbon fibre reinforced polymer material such as carbon fibre reinforced polyether ether ketone (CFR-PEEK). The shell may comprise fixation openings extending there through.

### Brief Description of the Drawings

For a better understanding of the present invention, and to show more clearly how it may be carried into effect, reference will now be made by way of example to the following drawings, in which:
Figure 1 is a perspective view of a liner, circlip and femoral head in an assembled and disassembled condition;
Figure 2 is a perspective view of an acetabular prosthesis component and femoral head prosthesis component;
Figure 3 is a sectional illustration of an acetabular shell; and
Figure 4 is a perspective view illustrating assembly of a hip prosthesis.

### Detailed Description of Embodiments

The present invention relates to a double mobility acetabular prosthesis component and cooperating femoral head prosthesis component that together provide a hip prosthesis having ceramic on ceramic and ceramic on carbon fibre reinforced polymer articulations.

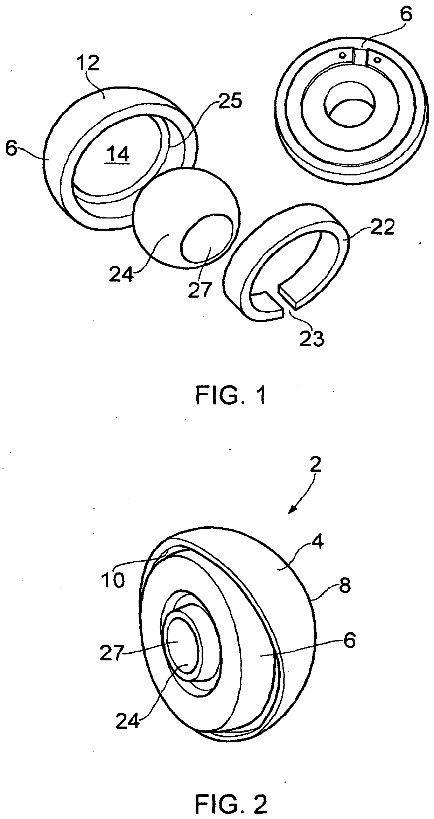

With reference to Figures 1 and 2, an acetabular prosthesis component 2 in accordance with an embodiment of the present invention comprises a shell 4 and a liner 6. The shell 4 is substantially hemispherical and is formed from a carbon fibre reinforced polymer material. According to the embodiment of the invention described below, the shell 4 is formed from carbon fibre reinforced polyether ether ketone (CFR-PEEK), however it will be appreciated that other fibre reinforced polymer materials may be considered. A carbon fibre reinforced polymer material such as CFR-PEEK provides a low wear bearing surface, as well as offering an alternative to metal bearing surfaces, concerns over which have been expressed in the literature owing to possible detrimental effects of metal ions released into the body from such surfaces.

The shell 4 is machined from bar or preferably injection moulded and comprises an outer surface 8, operable to engage with bone tissue when implanted into a patient, and an inner bearing surface 10. The inner bearing surface 10 is operable to receive the liner 6 in an articulating manner. The outer surface 8 may be provided with a hydroxyapatite (HA) coating 20 and/or a porous metal coating to stimulate bone in growth for additional fixation. The hydroxyapatite (HA) coating may be achieved by using thermal spraying (for example, using plasma spraying, arc spraying, flame spraying or using a high velocity oxyfuel), cold gas spraying or electrochemically assisted deposition. The porous coating may be achieved by using thermal spraying or cold gas spraying.

The liner 6 is also substantially hemispherical in shape and is formed from a ceramic material such as Alumina or Zirconia toughened Alumina. The liner comprises an outer bearing surface 12 that is operable to articulate with the inner bearing surface 10 of the shell. The liner also comprises an inner bearing surface 14, shaped to receive a femoral head prosthesis component, as described in further detail below. It will be appreciated that as the liner 6 is formed from a ceramic material, it is not necessary to form a lip on the shell 4 in order to support the liner 6 or to prevent creep deformation.

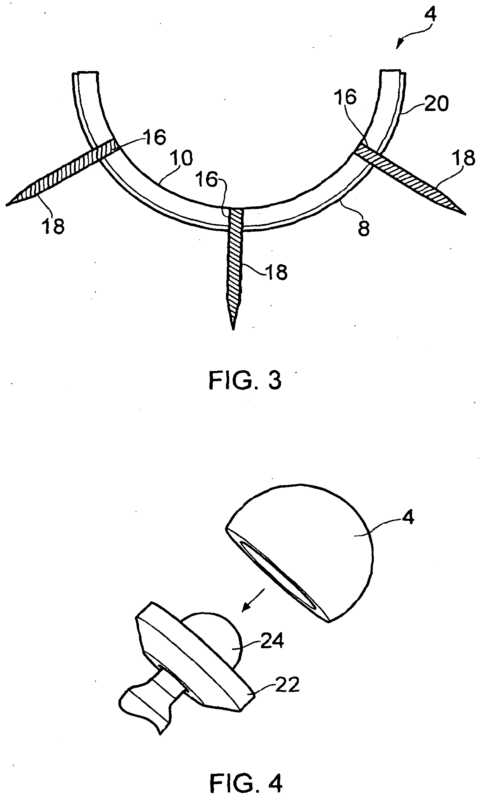

With additional reference to Figure 3, the shell 4 further comprises a plurality of openings 16 formed on the inner bearing surface 10 and extending through the thickness of the shell 4 to open onto the outer surface 8. The openings 16 may be clustered together or may be spaced across the bearing surface 10 as illustrated in the Figure. In one embodiment, the openings 16 comprise a series of substantially circular holes, sized to admit the passage of individual attachment means 18. One or more individual attachment means 18 is also provided as part of the prosthesis component. The attachment means pass through the openings 16 in the shell 4 and attach the shell 4 to a patient acetabulum, as described in further detail below. The attachment means may take the form of screws, pins, spikes, barbs or any other suitable attachment device. In the illustrated embodiment, the attachment means 18 take the form of screws but it will be appreciated that alternative attachment means may also be considered. The screws 18 are formed from a carbon fibre reinforced polymer material and in the present embodiment are formed from the same CFR-PEEK material as the shell 4, for example with a continuous fibre orientation. As the shell 4 and screws 18 are formed from the same CFR-PEEK material, the shell 4 presents a continuous inner bearing surface 10 and the presence of the screws 18 does not risk damaging the outer bearing surface 12 of the liner 6. In addition, CFR-PEEK operates under boundary lubrication, meaning the presence of the attachment means 18 does not affect the wear performance of the bearing. The articulation between the ceramic liner 6 and CFR-PEEK shell 4 is extremely low wear and hence desirable. In addition, the articulation contains no exposed metal, meaning that the production of metal ions is avoided.

Referring again to Figure 1, the prosthesis component 2 further comprises a circular clip, or circlip, 22 which is also formed from a carbon fibre reinforced polymer material, and in the present embodiment is formed from CFR-PEEK. The circlip 22 is of annular form and includes a split 23 to allow the component to be snapped into place over the femoral neck and engaging a distal portion of the femoral head, as described in further detail below. The circlip 22 is dimensioned to be received against an annular surface 25 of the liner 6, within the confines of the shell 4, as illustrated for example on Figure 1. The circlip 22 and liner 6 may comprise cooperating formations to enable the circlip 22 to be locked in place against the liner 6 once the femoral head is in position articulating against the inner bearing surface 14 of the liner 6.

Referring again to Figure 1, a prosthesis according to an embodiment of the present invention comprises the acetabular prosthesis component 2 described above and a femoral head prosthesis component 24. The femoral head prosthesis component 24 is a modular component, designed to cooperate with a stem and femoral neck prosthesis components in a known manner. For example the femoral head prosthesis component may comprise a recess 27 that forms one part of a Morse taper fixation arrangement, the recess being shaped to be received on a trunnion part of a femoral neck prosthesis component. The head 24 has a highly polished outer bearing surface designed to be received in an articulating manner against the inner bearing surface 14 of the liner 6. The head is formed from a ceramic material and according to the present embodiment is formed from the same ceramic material as the liner, which may for example be Alumina or Zirconia toughened Alumina. The hip prosthesis of the present invention thus provides a ceramic on ceramic articulation at the primary articulation of the joint, ceramic on ceramic being the lowest wearing material combination available.

Implantation and operation of the prosthesis will now be described with reference to the Figures. It will be understood that the details of the operative technique used may vary, including the order in which the femoral and acetabular prosthesis components are prepared and implanted. The following explanation is merely exemplary in nature, focussing on the issues specific to the prosthesis of the present invention.

Considering first the acetabular component, once the bone surface has been fully prepared, the shell 4 is implanted into the acetabulum of a patient. The level of fixation required in order to securely implant the shell 4 will vary according to the condition of the patient bone tissue. In some patients, there may be sufficient healthy bone tissue remaining in the acetabulum for the shell 4 to be implanted using simply a friction fit, or with the assistance of bone cement. In such cases, the openings 16 on the shell 4 and cooperating attachment means 18 may not be used. In order to maintain the physical continuity of the bearing surface of the shell 4, the openings 16 may be plugged using caps (not shown) formed from the same carbon fibre reinforced polymer material as the shell. Alternatively, the openings may be left uncovered in the implanted shell 4.

In patients experiencing significant degradation of acetabular bone tissue, there may not be sufficient healthy bone tissue available to enable a secure fixation using only friction and bone cement. In these cases, additional fixation is provided by the attachment screws 18, which are passed through the attachment openings 16 and into the surrounding bone tissue.

Once the shell is implanted into the patient acetabulum, the liner 6 may be inserted into the shell 4. As discussed above, the liner 6 and shell 4 articulate under boundary lubrication, which is not impacted by the presence of the CFR-PEEK screws.

The femoral prosthesis components, including stem, femoral neck and the femoral head 24 are prepared and assembled in a known manner, and a series of trial reductions may be carried out to ensure correct sizing and placement of components. Once the correct femoral head component 24 is correctly seated on the femoral neck and stem components, the head 24 is introduced into the liner 6, until the femoral head bearing surface articulates against the inner bearing surface 14 of the liner 6. The circlip 22 is then snapped around the femoral neck and into engagement with the liner 6 and shell 4 to hold the femoral head in place in engagement with the inner bearing surface 14 of the liner 6 (see Figure 4).

Once implanted, the principal articulation of the prosthesis is between the ceramic femoral head component 24 and the ceramic liner 6. At extremes of motion, the articulation between the ceramic liner 6 and the CFR-PEEK shell 4 is engaged. During articulation, the circlip 22 prevents micro separation or dislocation of the femoral head 24, as well as acting as a "bumper" to cushion the components.

The prosthesis of the present invention may be provided as a kit of parts, comprising shell 4, liner 6, circlip 22, attachment means 18 and femoral head 24 in a range of sizes, allowing a surgeon to select the most appropriate sizing combinations for a particular patient.

## Claims

1. A prosthesis component comprising: a shell (4) formed form a polymer material and having an inner bearing surface (10); and a liner (6) having an outer bearing surface formed from a ceramic material; wherein the liner is received in an articulating manner within the shell, **characterised in that** said polymer material is a fibre reinforced polymer material.

2. A prosthesis component as claimed in claim 1, wherein the shell further comprises one or more openings on the inner bearing surface.

3. A prosthesis component as claimed in claim 2, wherein the one or more openings extend through the thickness of the shell to open on an outer surface of the shell.

4. A prosthesis component as claimed in claim 2 or 3, wherein the openings are suitable to receive one or more attachment means operable to secure the prosthesis component in place.

5. A prosthesis as claimed in any one of claims 2 to 4, further comprising one or more attachment means, operable to extend through the openings to secure the prosthesis component in place.

6. A prosthesis component as claimed in claim 5, wherein the attachment means are formed from a fibre reinforced polymer material.

7. A prosthesis component as claimed in any one of the preceding claims, wherein the shell is formed from a carbon fibre reinforced polymer material.

8. A prosthesis component as claimed in any one of the preceding claims, wherein the shell is formed from carbon fibre reinforced polyether ether ketone (CFR-PEEK).

9. A prosthesis component as claimed in any one of the preceding claims, wherein a hydroxyapatite (HA) coating is formed on an outer surface of the shell.

10. A prosthesis component as claimed in any one of the preceding claims, wherein a porous metal coating is formed on an outer surface of the shell.

11. A prosthesis component as claimed in any one of the preceding claims, wherein the prosthesis component is an acetabular prosthesis component.

12. A prosthesis component as claimed in any one of the preceding claims, wherein the liner is formed from a ceramic material.

13. A prosthesis component as claimed in any one of the preceding claims, wherein the liner further comprises an inner bearing surface operable to receive a femoral head prosthesis component.

14. A prosthesis component as claimed in claim 13, wherein the component further comprises a retaining element, operable to cooperate with the liner and the shell to retain a femoral head prosthesis component in engagement with the inner bearing surface of the liner.

15. A prosthesis component as claimed in claim 14, wherein the retaining element is formed from a carbon fibre reinforced polymer material.

16. A prosthesis component as claimed in claim 14 or 15, wherein the retaining element comprises a circlip.

17. A kit of parts for a hip replacement prosthesis comprising: an acetabular shell (4) formed from a polymer material and comprising an inner bearing surface (10); an acetabular liner (6) and having an outer bearing surface formed from a ceramic material and operable to articulate with the inner bearing surface of the shell and an inner bearing surface; and a femoral head prosthesis having a bearing surface formed from a ceramic material and operable to articulate with the inner bearing surface of the liner, **characterised in that** said polymer material is a fibre reinforced polymer material.

## Patentansprüche

1. Prothesenkomponente, umfassend: eine Schale (4), die aus einem Polymermaterial gebildet ist und eine innere Lauffläche (10) aufweist; und eine Buchse (6), die eine aus einem Keramikmaterial gebildete äußere Lauffläche aufweist; wobei die Buchse auf gelenkige Weise in der Schale aufgenommen ist, **dadurch gekennzeichnet, dass** es sich bei dem Polymermaterial um ein faserverstärktes Polymermaterial handelt.

2. Prothesenkomponente nach Anspruch 1, wobei die Schale weiter eine oder mehrere Öffnungen an der inneren Lauffläche umfasst.

3. Prothesenkomponente nach Anspruch 2, wobei sich die eine oder die mehreren Öffnungen durch die Dicke der Schale erstrecken, um an einer äußeren Oberfläche der Schale zu öffnen.

4. Prothesenkomponente nach Anspruch 2 oder 3, wobei die Öffnungen geeignet sind, um ein oder mehrere Befestigungsmittel aufzunehmen, die wirksam sind, um die Prothesenkomponente ortsfest zu fixieren.

5. Prothesenkomponente nach einem der Ansprüche 2 bis 4, weiter umfassend ein oder mehrere Befestigungsmittel, die wirksam sind, um sich durch die Öffnungen zu erstrecken, um die Prothesenkomponente ortsfest zu fixieren.

6. Prothesenkomponente nach Anspruch 5, wobei die Befestigungsmittel aus einem faserverstärkten Polymermaterial gebildet sind.

7. Prothesenkomponente nach einem der vorangehenden Ansprüche, wobei die Schale aus einem kohlenstofffaserverstärkten Polymermaterial gebildet ist.

8. Prothesenkomponente nach einem der vorangehenden Ansprüche, wobei die Schale aus einem kohlenstofffaserverstärkten Polyetheretherketon (CFR-PEEK) gebildet ist.

9. Prothesenkomponente nach einem der vorangehenden Ansprüche, wobei eine Hydroxyapatit(HA)-Beschichtung auf einer äußeren Oberfläche der Schale gebildet ist.

10. Prothesenkomponente nach einem der vorangehenden Ansprüche, wobei eine poröse Metallbeschichtung auf einer äußeren Oberfläche der Schale gebildet ist.

11. Prothesenkomponente nach einem der vorangehenden Ansprüche, wobei es sich bei der Prothesenkomponente um eine Hüftgelenkpfannen-Prothesenkomponente handelt.

12. Prothesenkomponente nach einem der vorangehenden Ansprüche, wobei die Buchse aus einem Keramikmaterial gebildet ist.

13. Prothesenkomponente nach einen der vorangehenden Ansprüche, wobei die Buchse eine innere Lauffläche umfasst, die wirksam ist, um eine Hüftgelenkkopf-Prothesenkomponente aufzunehmen.

14. Prothesenkomponente nach Anspruch 13, wobei die Komponente weiter ein Halteelement umfasst, das wirksam ist, um mit der Buchse und der Schale zusammenzuwirken, um eine Hüftgelenkkopf-Prothesenkomponente im Eingriff mit der inneren Lauffläche der Buchse zu halten.

15. Prothesenkomponente nach Anspruch 14, wobei das Halteelement aus einem kohlenstofffaserverstärkten Polymermaterial gebildet ist.

16. Prothesenkomponente nach Anspruch 14 oder 15, wobei das Halteelement einen Sicherungsring umfasst.

17. Satz von Teilen für eine Hüftgelenkersatzprothese, umfassend: eine Hüftgelenkpfannenschale (4), die aus einem Polymermaterial gebildet ist und eine innere Lauffläche (10) umfasst; eine Hüftgelenkpfannenbuchse (6), die eine aus einem Keramikmaterial gebildete äußere Lauffläche, die wirksam ist, um mit der inneren Lauffläche der Schale ein Gelenk zu bilden und eine innere Lauffläche aufweist; und eine Hüftgelenkkopfprothese, die eine aus einem Keramikmaterial gebildete Lauffläche aufweist und wirksam ist, um mit der inneren Lauffläche der Buchse ein Gelenk zu bilden, **dadurch gekennzeichnet, dass** es sich bei dem Polymermaterial um ein faserverstärktes Polymermaterial handelt.

## Revendications

1. Composant de prothèse comprenant : une coquille (4) formée à partir d'un matériau polymère et comportant une surface intérieure d'appui (10) ; et un insert (6) comportant une surface extérieure d'appui, formé à partir d'un matériau céramique ; dans lequel l'insert est reçu de manière articulée à l'intérieur de la coquille, **caractérisé en ce que** ledit matériau polymère est un matériau polymère renforcé de fibres.

2. Composant de prothèse selon la revendication 1, dans lequel la coquille comprend en outre une ou plusieurs ouvertures sur la surface intérieure d'appui.

3. Composant de prothèse selon la revendication 2, dans lequel la ou les ouvertures s'étendent à travers l'épaisseur de la coquille pour déboucher sur une surface extérieure de la coquille.

4. Composant de prothèse selon la revendication 2 ou 3, dans lequel les ouvertures sont appropriées pour recevoir un ou plusieurs moyens de fixation pouvant servir à fixer en place le composant de prothèse.

5. Composant de prothèse selon l'une quelconque des revendications 2 à 4, comprenant en outre un ou plusieurs moyens de fixation pouvant servir à s'étendre à travers les ouvertures pour fixer en place le composant de prothèse.

6. Composant de prothèse selon la revendication 5, dans lequel les moyens de fixation sont formés à partir d'un matériau polymère renforcé de fibres.

7. Composant de prothèse selon l'une quelconque des revendications précédentes, dans lequel la coquille est formée à partir d'un matériau polymère renforcé de fibres de carbone.

8. Composant de prothèse selon l'une quelconque des revendications précédentes, dans lequel la coquille est formée à partir de poly(étheréthercétone) renforcée de fibres de carbone (CFR-PEEK).

9. Composant de prothèse selon l'une quelconque des revendications précédentes, dans lequel un revêtement d'hydroxy-apatite (HA) est formé sur une surface extérieure de la coquille.

10. Composant de prothèse selon l'une quelconque des revendications précédentes, dans lequel un revêtement métallique poreux est formé sur une surface extérieure de la coquille.

11. Composant de prothèse selon l'une quelconque des revendications précédentes, dans lequel le composant de prothèse est un composant de prothèse cotyloïdienne.

12. Composant de prothèse selon l'une quelconque des revendications précédentes, dans lequel l'insert est formé à partir d'un matériau céramique.

13. Composant de prothèse selon l'une quelconque des revendications précédentes, dans lequel l'insert comprend en outre une surface intérieure d'appui pouvant servir à recevoir un composant de prothèses de tête fémorale.

14. Composant de prothèse selon la revendication 13, dans lequel le composant comprend en outre un élément de retenue, pouvant servir à coopérer avec l'insert et la coquille pour retenir un composant de prothèse de tête fémorale en coopération avec la surface intérieure d'appui de l'insert.

15. Composant de prothèse selon la revendication 14, dans lequel l'élément de retenue est formé à partir d'un matériau polymère renforcé de fibres de carbone.

16. Composant de prothèse selon la revendication 14 ou 15, dans lequel l'élément de retenue comprend un circlip.

17. Ensemble de pièces de prothèse de remplacement de la hanche, comprenant : une coquille cotyloïdienne (4) formée à partir d'un matériau polymère et comprenant une surface intérieure d'appui (10) ; un insert cotyloïdien (6) et comportant une surface extérieure d'appui, formé à partir d'un matériau céramique et pouvant servir à assurer une articulation avec la surface intérieure d'appui de la coquille et une surface intérieure d'appui ; et une prothèse de tête fémorale comportant une surface d'appui formée à partir d'un matériau céramique et pouvant servir à assurer une articulation avec la surface intérieure d'appui de l'insert, **caractérisé en ce que** ledit matériau polymère est un matériau polymère renforcé de fibres.
